# FASCICULE DE BREVET EUROPEEN

(11) **EP 0 736 522 B1**
(45) Date de publication et mention de la délivrance du brevet: **29.12.1997**
(21) Numéro de dépôt: 96400546.6
(22) Date de dépôt: 15.03.1996
(51) Int. Cl.: C07C 233/18, C07C 233/20, C07C 235/08, A61K 7/48

(54) **Composés de type céramides, leur procédé de préparation et leur utilisation en cosmétique ou en dermatologie**
Ceramidverbindungen, Verfahren zu ihrer Herstellung und ihre Verwendung in der Kosmetik oder der Dermatologie
Ceramide compounds, process for their preparation and their use in cosmetics or dermatology

(30) Priorité: 05.04.1995 FR 9504050
(43) Date de publication de la demande: 09.10.1996
(73) Titulaire: L'OREAL, 75008 Paris (FR)
(72) Inventeur: Philippe, Michel, 91320 Wissous (FR); Luppi, Bernadette, 93270 Sevran (FR); Semeria, Didier, 77181 Courtry (FR)
(74) Mandataire: Tezier Herman, Béatrice

(56) Documents cités:
- EP-A- 0 500 437
- WO-A-93/20038
- US-A- 5 368 857
- JOURNAL OF ORGANIC CHEMISTRY, vol. 35, no. 2, 1970, EASTON US, pages 350-353, XP002008985 K. SISIDO ET AL.: "Syntheses of all of the racemic diastereoisomers of phytosphingosine"
- JOURNAL OF ORGANIC CHEMISTRY, vol. 34, no. 11, 1969, EASTON US, pages 3539-3544, XP002008986 K. SISIDO ET AL.: "Synthesis of racemic phytosphingosine and the lyxo isomer"
- CHEM. PHYS. LIPIDS (1979), 23(4), 291-319, XP000576829 KULMACZ, RICHARD J. ET AL: "Sphingolipid base metabolism. Chemical syntheses and properties of N-acetyl derivatives of 4R-, 4S-, 5R-, and 5S-hydroxysphinganine"
- TETRAHEDRON LETT. (1981), 22(44), 4429-32, XP002008987 MORI, KENJI ET AL: "Synthesis of both 2,3-erythro- and 2,3-threo- isomers of aplidiasphingosine, a bioactive marine terpenoid"
- CHEMICAL ABSTRACTS, vol. 121, no. 15, 10 Octobre 1994 Columbus, Ohio, US; abstract no. 175530, BERLINCH, ROBERTO GOMES DE SOUZA: page 665; XP002008989 & QUIM. NOVA, vol. 17, no. 2, 1994, pages 167-171,
- CHEMICAL ABSTRACTS, vol. 117, no. 3, 20 Juillet 1992 Columbus, Ohio, US; abstract no. 25005, KOJIMA, MICHIYUKI ET AL.: page 565; XP002008990 & YUKAGAKU, vol. 41, no. 3, 1992, pages 252-7,
- CHEMICAL ABSTRACTS, vol. 115, no. 17, 28 Octobre 1991 Columbus, Ohio, US; abstract no. 179361, KOJIMA, MICHIYUKI ET AL.: page 537; XP002008991 & J. AGRIC. FOOD CHEM., vol. 39, no. 10, 1991, pages 1709-14,
- LIEB. ANN. CHEM., 1991, pages 125-128, XP002008988 R. KRAUS, G. SPITELLER: "Ceramides from urtica dioica roots"
- CHEMICAL ABSTRACTS, vol. 112, no. 9, 26 Février 1990 Columbus, Ohio, US; abstract no. 73760, OHNISHI ET AL.: page 474; XP002008992 & YUKAGAKU , vol. 38, no. 8, 1989, pages 647-53,
- CHEMICAL ABSTRACTS, vol. 103, no. 5, 5 Août 1985 Columbus, Ohio, US; abstract no. 35441, S. A. LONG: page 337; XP002008993 & ARCH. DERMATOL. RES., vol. 277, no. 4, 1985, pages 284-7,
- CHEMICAL ABSTRACTS, vol. 75, no. 13, 27 Septembre 1971 Columbus, Ohio, US; abstract no. 85024, K. L. KARLSSON ET AL.: page 78; XP002008994 & J. LIPID RES., vol. 12, no. 4, 1971, pages 466-472,
- CHEMICAL ABSTRACTS, vol. 122, no. 21, 22 Mai 1995 Columbus, Ohio, US; abstract no. 261714, ROBSON, KRISTI J. ET AL: "6-Hydroxy-4-sphingenine in human epidermal ceramides" XP002008995 & J. LIPID RES. (1994), 35(11), 2060-8,

## Description

La présente invention a pour objet de nouveaux composés de type céramides, leur procédé de préparation ainsi que leur utilisation, notamment pour les traitements et les soins de la peau, des cheveux, des ongles et des cils en cosmétique ou en dermatologie.

L'exposition de la peau au froid, au soleil, aux atmosphères à faible humidité relative, les traitements répétés avec des compositions de lavage ou encore le contact avec des solvants organiques, sont des facteurs qui entraînent, à des degrés divers, un déssèchement apparent. La peau apparaît plus sèche, moins souple et le relief cutané plus prononcé. Par ailleurs, les cheveux, qui sont soumis trop fréquemment à certains traitements capillaires, perdent leur aspect brillant et peuvent devenir rèches et cassants.

La demanderesse a donc recherché des composés qui permettent de prévenir ou de corriger ces phénomènes se traduisant par un déssèchement apparent et qui redonnent à la peau sa souplesse et aux cheveux leur brillance et leur douceur.

Pour résoudre ce problème, on a déjà proposé d'utiliser des céramides. On sait en effet que ces composés sont les éléments constitutifs prépondérants des lipides interconéocytaires du stratum cornéum et participent au maintien de l'intégrité de la barrière cutanée.

Les céramides utilisés en cosmétique sont le plus souvent des extraits naturels issus notamment de la peau de porc, du cerveau de boeuf, de l'oeuf, des cellules de sang, des végétaux comme le blé, etc. (demandes de brevets japonais J 86/260008 et J 87/120308). De tels céramides ont été également proposés pour la protection des cheveux (EP 0 278 505).

Il s'agit donc toujours de mélanges de teneur plus ou moins importante en céramides et dont la composition est difficile à contrôler. De plus, ces mélanges sont sujets à la contamination bactérienne. Leur conservation est donc difficile à maîtriser. Lorsqu'ils sont d'origine animale, il y a en plus un risque de contamination par l'agent responsable de la BSE (encéphalopathie bovine spongiforme).

Chaque céramide d'origine naturelle présente une stéréoisomérie précise et unique, telle que celles décrites pour la sphinganine, la phytoshingosine et la sphingosine (encore appelée la sphingénine) qui sont respectivement le (*2S,3R*)-2-amino-1,3-octadécanediol, le (*2S*,*3S*,*4R*)-2-amino-1,3,4-octadécanetriol et le (*2S*,*3R*,*4E*)-2-amino-4-octadécène-1,3-diol [J. Biochem. 79, 11-21 (1977)].

Pour résoudre ces problèmes, nous avons proposé des céramides de synthèse, notamment dans la demande de brevet européen n° 0 500 437. Ces composés, utilisés dans des compositions cosmétiques ou dermatologiques, pour les traitements et les soins de la peau et des cheveux ont un effet hydratant permettant de prévenir ou de corriger certains effets du déssèchement apparent de la peau ou des cheveux.

Toutefois, il serait souhaitable de mettre au point des composés qui, utilisés dans des compositions cosmétiques ou dermatologiques, aient un effet hydratant ou traitant supérieur à celui des composés de cette demande de brevet.

Il faut également noter une demande brevet WO 94/10131 décrivant un procédé de fermentation qui conduit à une famille de céramides apparentés aux céramides 1 dans la classification de DOWNING (J.I.D.-84, 410-412,1985), ce procédé est donc malheureusement restreint à cette famille de céramides.

La présente invention a donc pour objet des composés répondant à la formule : 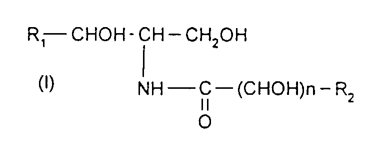 dans laquelle :
* R₁ désigne un radical alkyle hydroxylé, saturé ou insaturé, en C₁₀ à C₂₅ ;
* n est égal à 0 ou 1 ;
* R₂ désigne un radical alkyle linéaire ou ramifié, saturé ou insaturé, en C₁ à C₃₁, lorsque n égal 1; R₂ désigne un radical alkyle linéaire ou ramifié, saturé ou insaturé, en C₂ à C₃₁, lorsque n égal 0 ;
les composés étant sous forme de mélanges d'isomères au moins sur la partie amino-alcool de ladite formule (I).

Ainsi, ces nouveaux composés présentent un très bon pouvoir hydratant de la peau et/ou des cheveux lorsqu'ils sont utilisés dans des compositions cosmétiques ou dermatologiques.

Lorsque R₁ désigne un radical alkyle saturé et hydroxylé, on préfère que le radical hydroxyle se trouve en position α par rapport au carbone du groupement -CHOH-. Ce radical hydroxyle peut se trouver sous une forme subsituée. Ce radical peut alors être représenté notamment par un radical de formule -O-CO-CHOH-R₄, avec R4 désignant un radical alkyle linéaire ou ramifié, saturé ou insaturé, en C₁ à C₃₁.

Lorsque R₁ désigne un radical alkyle insaturé et hydroxylé, on préfère que R₁ présente un radical éthylénique en position α par rapport au carbone du groupement -CHOH-. Plus particulièrement, au moins un, et de préférence le, radical hydroxyle est en position α du radical éthylénique.

De préférence, R₁ désigne un radical alkyle saturé et hydroxylé.

De préférence, R₁ désigne un radical alkyle hydroxylé, saturé ou insaturé, en C₁₂ à C₂₃.

De préférence, R₂ désigne un radical alkyle linéaire, plus particulièrement en C₂ à C₂₅.

Les composés selon l'invention sont donc sous forme de mélanges d'isomères optiques et/ou géométriques (mélange d'énantiomères et/ou de diastéréoisomères) au moins sur la partie amino-alcool, ce qui les différencient des produits naturels qui sont uniquement sous forme d'un seul isomère.

Ces nouveaux composés ont l'avantage d'améliorer et/ou de rétablir la fonction barrière lorsqu'ils sont appliqués sur la peau.

Les composés selon l'invention sont de préférence sous forme de mélange d'au moins 4 isomères.

De préférence, les composés de formule (1) sont choisis parmi le 2-N-docosanoylaminooctadécane-1,3,4-triol, le 2-N-(2-hydroxy-hexadecanoyl)-amino-octadécane-1,3,4-triol et le 2-N-hexadecanoylamino-octadécane-1,3,4-triol.

Les cheveux traités par ces composés de formule (I) présentent un aspect brillant, un toucher plus doux et une moins grande sensibilité à l'eau, due à l'apport de matière lipidique uniformément répartie sur les écailles du cheveu. Les propriétés mécaniques et de nervosité sont également améliorées.

Les composés selon l'invention peuvent former avec d'autres lipides des vésicules.

Les composés selon l'invention peuvent être obtenus par acylation de la fonction amine des composés de formule (II) ou de dérivés réactifs de ceux-ci, tel que par exemple le chlorhydrate, avec un agent acylant approprié.

Ainsi, un autre objet de l'invention concerne un procédé de préparation de mélanges d'isomères des composés de formule (I) ci-dessus, caractérisé en ce qu'il comprend :
(a) l'acylation d'au moins un composé de formule (II) suivante : 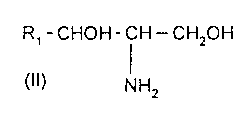 sous forme de mélange d'au moins 2 isomères,
   formule (II) dans laquelle R₁ a la même signification. que donnée ci-dessus, ou d'au moins un dérivé réactif de ces composés de formule (II), à l'aide d'au moins un agent acylant de formule (III) suivante :

   R₂-(CHX)ₙ-CO-A (III)

   formule (III) dans laquelle R₂ et n ont les significations données précédemment et X représente un groupe choisi parmi le radical hydroxyle, un atome d'halogène, de préférence Br, Cl ou l, et un radical de formule OB susceptible de former un groupe OH ; A est un groupe choisi parmi un atome d'halogène, un radical de formule -O-COO-R₅, un radical de formule OR₆, un groupement 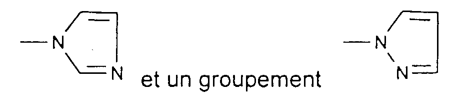 où R₅ est un radical alkyle inférieur en C₂ à C₈ et R₆ est un groupe choisi parmi un radical alkyle inférieur en C₁ à C₈, 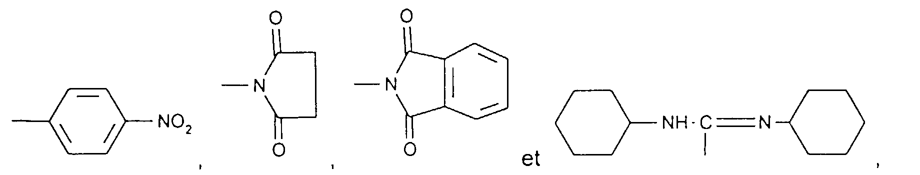
(b) l'isolation des composés obtenus, et
(c) éventuellement, lorsque X est différent du radical hydroxyle, l'hydrolyse ou l'hydrogénolyse des composés obtenus pour transformer le groupe OB en radical hydroxyle, précédé éventuellement, lorsque X est un atome d'halogène, d'une étape de substitution de X par un groupe OB.

Ainsi, par partie amino-alcool, on entend selon l'invention la partie de la formule (I) venant du composé de formule (II).

De préférence, le procédé est réalisé en présence d'un solvant approprié. Parmi les solvants utilisables dans le procédé de la présente invention, on peut citer le tétrahydrofurane (THF), la pyridine, le 1,2-diméthoxy-éthane, le diméthylformamide, le dichlorométhane, le tertiobutylméthyléther.

De préférence, le groupement OB est choisi parmi les radicaux suivants : acétate, benzoate, benzyloxy, -OSi(CH₃)₃, -OSi(CH₃)₂(t-butyl), et -OSi(t-butyl)(-C₆H₅)₂. De manière encore plus préférentielle, OB est un groupement acétate.

De préférence, A est un groupe choisi parmi les groupes suivants :
-Cl, un radical de formule -O-COO-C₂H₅, un radical de formule -OCH₃ ou -OC₂H₅,

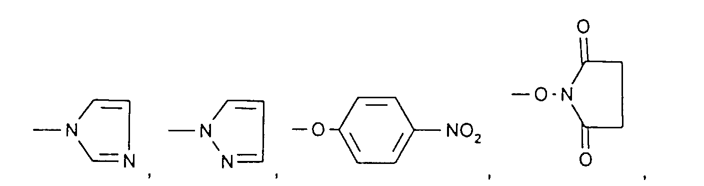

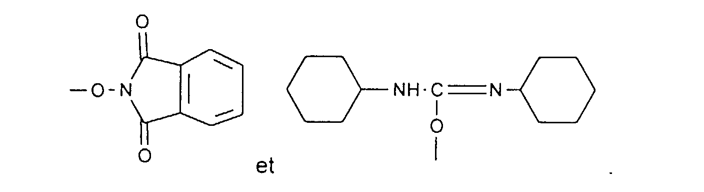

On recommande tout particulièrement pour A les radicaux

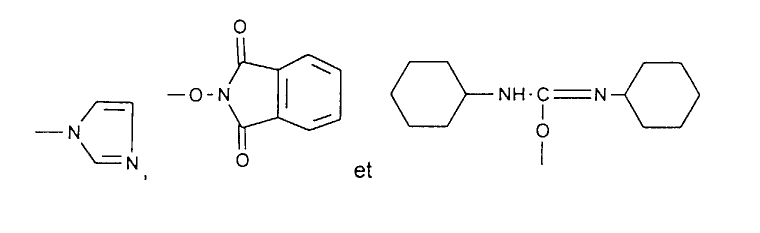

Les agents acylants particulièrement recommandés sont les esters de succinimide et de carbodiimide, les dérivés imidazoles et les chlorures d'acide, tels que définis ci-dessus.

Pour la préparation des composés de formule (I), on peut donc également utiliser des sels, tels que les chlorhydrates des composés de formule (II).

Les composés de formule (II) ou leurs dérivés sont connus et ont été notamment décrits par M. Prostenik (Croatia Chemica Acta, 29, 107-113, 1957) et M. Jäger (Angew. Chem. Int., 603-605, 1981). Ces composés sont donc des mélanges d'au moins 2 isomères optiques et/ou géométriques (énantiomères et/ou diastéréoisomères).

Les agents acylants de formule (III) sont des produits connus de l'homme du métier, ils peuvent être également sous forme de mélanges d'isomères, notamment de mélanges racémiques.

Généralement, les quantités des composés de formule (II) et (III) mis en oeuvre dans le procédé selon l'invention sont choisies de telle sorte que leur rapport molaire (III)/(II) est supérieur ou égal à 1.

La température du procédé selon l'invention peut varier dans une large mesure. La température de l'étape (a) est généralement comprise entre 0 et 50°C, de préférence, elle correspond à la température ambiante.

Avant l'étape (b) du procédé selon l'invention et lorsque X représente le radical hydroxyle, les produits de l'étape (a) peuvent subir une réaction de protection des groupements hydroxyle, par réaction avec un agent choisi parmi les anhydrides d'acide, les halogénures d'acide et les chlorosilanes, la réaction étant suivie, après isolement des composés selon l'étape (b), par une hydrolyse ou une hydrogénolyse.

Les agents utilisés dans le procédé de la présente invention sont de préférence choisis parmi l'anhydride acétique, le chlorure de benzoyle, le chlorure de benzyle, le bromure de benzyle, les chlorosilanes de formule ClSi(CH₃)₃, ClSi(CH₃)₂(tBu), ClSi(tBu)(-C₆H₅)₂.

Les composés selon l'invention peuvent recevoir des applications diverses, notamment dans des compositions cosmétiques et dermatologiques. Ces composés possèdent en plus la propriété de former des vésicules en association avec d'autres lipides, lorsqu'ils sont dispersés dans l'eau.

La présente invention a donc pour objet l'utilisation des composés de formule (I) dans des émulsions, des dispersions ou dans des lotions. Elle a également pour objet l'utilisation de ces composés, associés à d'autres lipides, pour la formation de sphérules lipidiques.

La présente invention a également pour objet des compositions à usage cosmétique ou dermatologique contenant les composés de formule (I).

Un autre objet de l'invention est un procédé de traitement cosmétique de la peau, des cheveux, des ongles ou des cils consistant à appliquer sur ces derniers une quantité suffisante d'au moins un composé de formule (I).

Les compositions selon l'invention peuvent se présenter sous forme d'émulsions (lait ou crème), de lotions hydroalcooliques, huileuses ou oléoalcooliques, de gels, de dispersions ou de bâtonnets solides, de sprays ou de mousse aérosol.

Selon l'invention, les composés de formule (I) représentent de 0,005% à 20%, de préférence de 0,01 à 10% du poids total de la composition.

Les compositions sont par exemple des lotions, des laits ou des crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings, des rouges à lèvres, des mascaras ou des vernis à ongles.

Ces compositions peuvent également se présenter sous la forme de bâtons pour les lèvres destinés soit à les colorer, soit à éviter les gerçures, ou de produits de maquillage pour les yeux ou des fards et fonds de teint pour le visage.

Lorsque les compositions selon l'invention se présentent sous la forme d'émulsions de type eau-dans-l'huile ou huile-dans-l'eau, la phase grasse est essentiellement constituée d'un mélange de composés de formule (I) avec au moins une huile, et éventuellement un autre corps gras.

La phase grasse des émulsions peut constituer de 5 à 60% du poids total de l'émulsion.

La phase aqueuse desdites émulsions constitue de préférence de 30 à 85% du poids total de l'émulsion.

La proportion de l'agent émulsionnant peut être comprise entre 1 et 20%, et de préférence entre 2 et 12% du poids total de l'émulsion.

Lorsque les compositions selon l'invention se présentent sous forme de lotions huileuses, oléoalcooliques ou hydroalcooliques, elle peuvent constituer, par exemple, des lotions antisolaires contenant un filtre absorbant les rayons UV, des lotions adoucissantes pour la peau; les lotions huileuses peuvent en outre constituer des huiles moussantes contenant un tensio-actif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acide gras tels que les triglycérides d'acides gras ayant de 6 à 18 atomes de carbone, les alcools gras; les émulsionnants comme les alcools gras éthoxyéthylénés ou les alcoyléthers de polyglycérol; les solvants tels que les monoalcools ou polyalcools inférieurs contenant de 1 à 6 atomes de carbone ou encore l'eau.

Les mono- ou poly-alcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, de germes de blé, d'olive, de germe de maïs, de jojoba, de sésame, de tournesol, de palme, de noix, de karité, de shoréa, de macadamia, de pépins de cassis et similaires.

Parmi les esters d'acides gras, on peut utiliser des esters d'acides en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles'hydrogénées concrètes à 25°C, les sucroglycérides, les oléates, myristates, linoléates et stéarates de calcium, magnésium et aluminium.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C₁₂-C₁₈ comportant de 2 à 10 moles de glycérol.

Il peut être aussi utile d'utiliser des épaississants tels que les dérivés de cellulose, les dérivés d'acide polyacrylique, les gommes de guar ou de caroube ou la gomme de xanthane.

La composition selon l'invention peut également contenir des adjuvants habituellement utilisés en cosmétique ou en dermatologie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de composés de formule (I) dans l'eau en présence de tensio-actif ou encore de dispersions aqueuses de sphérules lipidiques, constituées de couches moléculaires organisées enfermant une phase aqueuse encapsulée, ces couches étant constituées d'au moins un mélange d'isomères de composés de formule (I) associé à au moins un autre composé lipidique.

On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991 , 1 477 048 et 2 091 516 ou dans les demandes de brevet international WO 83/01 571 et WO 92/08685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tels que ceux décrits dans les brevets français n° 1 477 048, 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

La présente invention a donc pour objet une dispersion de sphérules lipidiques constituées de couches moléculaires organisées de composés de formule (I) et de lipide défini ci-dessus renfermant une phase aqueuse à encapsuler.

La phase continue de la dispersion qui entoure les sphérules est une phase aqueuse.

Les sphérules en dispersion ont généralement un diamètre compris entre 0,05 µm et 5 µm.

La phase aqueuse encapsulée dans les sphérules peut être de l'eau ou une solution aqueuse de substance active et est dans ce cas de préférence isoosmotique par rapport à la phase continue de la dispersion.

Les sphérules peuvent être obtenues en particulier suivant le procédé décrit dans le brevet français 2 315 991 de la Demanderesse, selon lequel on prépare une dispersion de sphérules constituées de couches moléculaires organisées renfermant une phase aqueuse à encapsuler, en mettant en contact d'une part des mélanges d'isomères de composés de formule (I) associées à un ou plusieurs lipide(s) défini(s) ci-dessus et d'autre part la phase aqueuse à encapsuler dans les sphérules, en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.

Un autre procédé de préparation peut consister à utiliser le procédé dénommé REV (reverse-phase evaporation vesicle) ou évaporation en phase inverse décrit dans Proc. Natl. Acad. Sci. USA., Vol. 75, n° 9, pages 4194-4198 (1978), par SZOKA et PAPAHADJOPOULOS.

On peut également mettre en oeuvre le procédé qui comprend la succession d'étapes consistant à dissoudre au moins un lipide dans au moins un solvant organique non miscible à l'eau ; ajouter la phase organique ainsi obtenue à une phase aqueuse ; former une dispersion des deux phases sous forte agitation, la taille des vésicules pouvant être réglée en faisant varier la vitesse d'agitation au cours de ce mélange de phase ; conduire l'évaporation du (ou des) solvant(s) sous forte agitation ; et, le cas échéant, concentrer la dispersion.

Les substances actives peuvent être des substances ayant un intérêt pharmaceutique, alimentaire ou des substances ayant une activité cosmétique. Lorsqu'elles sont hydrosolubles, elles sont dans la phase aqueuse encapsulée à l'intérieur des vésicules.

Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels; des agents de brunissage artificiel tels que la dihydroxyacétone, l'érythrulose, le glycéraldéhyde, les γ-dialdéhydes tels que l'aldéhyde tartrique, ces composés étant éventuellement associés à des colorants ; des filtres solaires hydrosolubles; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines, les hormones, les enzymes telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles se trouvent incorporées dans les feuillets des vésicules. Elle peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

Les dispersions de sphérules lipidiques présentent l'intérêt de véhiculer des substances actives qui se trouvent ainsi masquées et protégées vis-à-vis des différents agents d'altération : oxydants et plus généralement composés réactifs vis-à-vis des substances actives encapsulées. La pénétration et la fixation des substances actives peuvent être modulées par la variation de la taille des sphérules et de leur charge électrique. L'action de ces substances actives peut également être ainsi différée (effet retard).

L'invention a enfin pour objet l'utilisation en cosmétique d'une dispersion aqueuse de sphérules constituée de couches moléculaires organisées de composés de formule (I) associés à d'autres lipides renfermant une phase aqueuse à encapsuler, en particulier pour le traitement de la peau.

L'invention a également pour objet l'utilisation d'une telle dispersion de sphérules lipidiques en dermatologie ou dans l'industrie alimentaire.

Dans ce qui suit ou ce qui précède, les pourcentages sont donnés en poids, sauf mention contraire.

Les exemples qui suivent sont donnés à titre illustratif et non limitatif.
THF signifie tétrahydrofurane, M.A. signifie matière active.

### EXEMPLE 1

### Préparation du 2-N-hexadecanoylamino-octadécane-1,3,4-triol (2 isomères D,L-ribo)

Le 2-amino-octadécane-1,3,4-triol (2 isomères D,L-ribo, 450mg, 1,4.10⁻³ mole) est mis en suspension dans 25ml de tétrahydrofurane (THF). On ajoute en une fois le chlorure de palmitoyle (390mg, 1,4.10⁻³ mole). La triéthylamine (145mg, environ 1,4.10⁻³ mole) est coulée lentement. Au bout de 4 heures, il ne reste plus d'amine. Par addition d'eau, dans le milieu réactionnel on obtient un précipité.

Après filtration, lavage et séchage, on obtient 730mg de solide blanc (rendement : 94%).

Le solide est recristallisé dans du méthanol et du THF, on récupère alors 400 mg de cristaux blancs (rendement : 51%). Le spectre RMN 13C, la spectrographie de masse et l'analyse élémentaire du produit obtenu sont conformes au produit attendu de point de fusion de 124-125°C :
le 2-N-hexadecanoylamino-octadécane-1,3,4-triol (2 isomères D,L-ribo)

### EXEMPLE 2

### Préparation du 2-N-(2-hydroxy-hexadecanoyl)-amino-octadécane-1,3,4-triol (4 isomères)

L'acide D,L-2-hydroxy-hexadécanoïque (2g - 7,5.10⁻³ mole) est mis en suspension dans 50ml d'actérate d'éthyle. On ajoute rapidement du N-hydroxysuccinimide (0,8g - 7,5.10⁻³ mole) et du dicyclohexylcarbodiimide (1,5g - 7,5.10⁻³ mole). On agite 2 heures à température ambiante. On filtre la dicyclohexylurée. Le solide obtenu est remis en suspension dans 20 ml de THF et cette solution est coulée dans un mélange, 2-aminooctadécane-1,3,4-triol (2 isomères D,L-ribo - 2,4g - 7,5.10⁻³ mole) et 100ml de THF, la solution est portée à reflux. Après 1 heure de reflux, la réaction est arrêtée et on laisse revenir à température ambiante. On ajoute directement dans le milieu réactionnel 50g de silice.

Après filtration et évaporation à sec, on obtient 3,8 g de solide blanc (rendement 88%). On recristallise le produit obtenu dans un mélange éthanol/eau (9/1). On obtient alors 2,1 g de cristaux blancs (rendement 49%). Le spectre RMN 13C et l'analyse élémentaire du produit obtenu sont conformes au produit attendu de point de fusion de 134-136°C : le 2-N-(2-hydroxy-hexadecanoyl)-amino-octadécane-1,3,4-triol (4 isomères).

### EXEMPLE 3

### Préparation du 2-N-docosanoylamino-octadécane-1,3,4-triol (2 isomères D,L-ribo)

L'acide béhénique (530mg - 1,6.10⁻³ mole) est mis en solution dans 10ml d'acétate d'éthyle. On ajoute rapidement le N-hydroxysuccinimide (175mg - 1,6.10⁻³ mole) et le dicyclohexyl-carbodiimide (325mg - 1,6.10⁻³ mole). On agite à température ambiante pendant 2 heures, on filtre la dicyclohexylurée et on évapore à sec.
Le solide obtenu est remis en solution dans 5ml de THF et coulé rapidement dans une solution de 2-amino-octadécane-1,3,4 - triol (2 isomères D,L-ribo - 500mg - 1,6.10⁻³ mole - 5ml de THF) au reflux. Ce reflux est maintenu 1 heure.

On purifie par chromatographie, le milieu réactionnel étant déposé directement (l'éluant étant un mélange de dichloro1,2-éthane et d'isopropanol (80/20)). On isole ainsi 150 mg de solide blanc. Le spectre RMN 13C et l'analyse élémentaire du produit obtenu sont conformes au produit attendu de point de fusion de 123-124°C :
2-N-docosanoylamino-octadécane-1,3,4-triol (2 isomères D,L-ribo)

### EXEMPLE 4

### Préparation du 2-N-hexadécanoylamino-octadécane-1,3,4-triol (8 isomères)

0,5g de chlorhydrate de 2-amino-octadécane-1,3,4-triol (8 isomères - 1,4.10⁻³ mole) sont mis en solution dans 25 ml de THF. On ajoute rapidement un équivalent de triéthylamine, puis 0,39g de chlorure de palmitoyle (1,4.10⁻³ mole). On termine par l'addition d'un deuxième équivalent de base.

Après une agitation de 30 minutes à température ambiante et par addition de deux volumes d'eau, on obtient un précipité blanc. Ce précipité est filtré, lavé avec de l'eau puis séché sous vide en présence de P₂O₅.

Après recristallisation dans de l'acétate d'éthyle, on obtient 0,4g de poudre blanche. (rendemant 78%). Le spectre RMN 13C et l'analyse élémentaire du produit obtenu sont conformes au produit attendu :
2-N-hexadécanoylamino-octadécane-1,3,4-triol (8 isomères)

### EXEMPLE 5

### Préparation du 2-N-(2-hydroxy-hexadécanoyl)-amino-octadécane-1,3,4-triol (16 isomères)

- Procédé A
   2g d'acide D,L-2-hydroxyhexadécanoïque (7,5.10⁻³ mole) sont mis en solution dans 50ml d'acétate d'éthyle. On ajoute rapidement 0,8g de N-hydroxysuccinimide (7,5.10⁻³ mole) et 1,5g de dicyclohexylcarbodiimide (7,5.10⁻³ mole). On agite à température ambiante pendant 2 heures. On filtre la dicyclohexylurée et on concentre la solution sous vide. On redissout le solide ainsi obtenu dans 20ml de THF. On coule cette solution dans un réacteur contenant 2,4g de 2-amino-octadécane-1,3,4-triol ( 8 isomères - 7,5.10⁻³ mole) en solution dans 100ml de THF au reflux. Après une heure de reflux, on évapore à sec.
   On purifie le produit ainsi obtenu par chromatographie sur colonne de silice (l'éluant étant : dichloro-1,2-éthane (9)/méthanol(1)). 1g de solide blanc est isolé. Le spectre de masse du produit obtenu correspond à la structure attendue de point de fusion de 138-140°C : 2-N-(2-hydroxy-hexadécanoyl)-amino-octadécane-1,3,4-trio (16 isomères)
- Procédé B
   37g de 2-amino-octadécane-1,3,4-triol (8 isomères - 1,16.10⁻¹ mole) sont mis en solution dans 300ml de THF. Quand le milieu est homogène, on refroidit vers 20°C. On ajoute rapidement 41,3g de chlorure d'acide de l'acide D,L-2-bromohexadécanoïque (1,16.10⁻¹ mole) et 16,5ml de triéthylamine (1,16.10⁻¹ mole). On chauffe à 50°C pendant 2 heures. On verse l'ensemble dans 2 litres d'eau glacée sous agitation. On laisse sous agitation pendant 1 heure. On filtre, on lave à l'eau puis on sèche.
   On met ensuite en solution 73g de dérivé bromé obtenu (1,15.10⁻¹ mole) dans 250ml de N-méthylpyrrolidone. On ajoute 22,5g d'acétate de potassium (2,3.10⁻¹ mole). On chauffe à 90°C pendant 4 heures. On refroidit et on verse dans 2 litres d'eau glacée. On filtre rapidement, on relave, puis on sèche.
   46g d'acétate obtenu (7,3. 10⁻² mole) sont mis en solution dans 350ml de méthanol chaud. On laisse revenir à température ambiante et on ajoute 2,2ml d'une solution de méthylate de sodium à 30% dans le méthanol. Le milieu réactionnel est agité pendant 4 heures. On filtre, on sèche et recristallise dans le minimum d'heptane. On obtient 20g de solide très légèrement beige. L'analyse élémentaire du produit obtenu est conforme au produit attendu de point de fusion de 138-140°C (produit équivalent au produit obtenu par le procédé A) :
   2-N-(2-hydroxy-hexadécanoyl)-amino-octadécane-1,3,4-trio (16 isomères)

### EXEMPLE 6

Cette exemple comprend différentes formulations pour le soin ou le traitement des cheveux avec des composés des exemples précédents

| **Shampooing** | |
|---|---|
| Lauryl éther sulfate de sodium (28%M.A.) | 75 g |
| Monoisopropanolamide d'acide de coprah | 1 g |
| vendu par Albright et Wilson sous le nom d'Empilan CIS | |
| Composé de l'exemple 5 | 1 g |
| Eau q.s.p. | 100 g |

Le shampooing ainsi formulé est d'aspect limpide

| **Soin après-shampooing à rincer** | |
|---|---|
| Méthosulfate de 1-méthyl 2-suif 3-surfamido | 2 g M.A. |
| éthylimidazolium/propylène glycol (75/25) | |
| vendu par Witco sous le nom de Rewoquat W75PG | |
| composé de l'exemple 5 | 0,5 g |
| Mélange d'alcool cétylique | 3 g |
| et cétyl stéarylique oxyéthyléné | |
| Conservateur, parfum | |
| Eau q.s.p. | 100 g |
| pH spontané de 5,2 | |

| **Shampooing** | |
|---|---|
| Lauryl éther sulfate de sodium (28% M.A.) | 60 g |
| Cocoylbétaïne | 9 g |
| Composé de l'exemple 4 | 0,5 g |
| Conservateur, parfum | |
| Eau q.s.p. | 100 g |
| HCl q.s. | pH 6 |

Le shampooing ainsi formulé est opalescent.

| **Lotion démêlante à rincer** | |
|---|---|
| Chlorure de béhényltriméthylammonium à 80% | 0,5 g M.A. |
| dans un mélange eau/iropropanol (15/85) | |
| vendu par Toho (Catinal DC50®) | |
| Composé de l'exemple 4 | 0,1 g |
| Conservateur, parfum | |
| Eau q.s.p. | 100 g |
| NaOH q.s. | pH 5,5 |

### EXEMPLE 7

Cette exemple comprend différentes formulations pour le soin ou le traitement de la peau avec des composés des exemples précédents

| **Emulsion huile dans eau hydratante** | |
|---|---|
| Huile de germe de maïs | 2 g |
| Monostéarate de glycérol | 3 g |
| Polyéthylène glycol 400 | 3 g |
| Carbopol 941 ® vendu par Goodrich | 0,2 g |
| Myristate d'isopropyle | 3,0 g |
| Composé de l'exemple 4 | 0,1 g |
| Alcool cétylique | 3,0 g |
| Alcool stéarique | 3,0 g |
| NaOH | 0,008 g |
| Propylène glycol | 5,0 g |
| Conservateurs | 0,3 g |
| Eau q.s.p. | 100 g |

| **Emusion eau dans l'huile hydratante** | |
|---|---|
| Huile de vaseline | 10,0 g |
| Protegin X vendu par Goldschmidt | 20,0 g |
| Huile de tournesol | 15,0 g |
| Composition aromatique | 1,0 g |
| Composé de l'exemple 5 | 0,05 g |
| Sulfate de magnésium | 0,5 g |
| Glycérol | 5,0 g |
| Cétrol HE vendu par Henkel | 4,0 g |
| Conservateurs | 0,3 g |
| Eau q.s.p. | 100 g |

| **Gel aqueux** | |
|---|---|
| Carbopol 940® vendu par Goodrich | 0,6 g |
| Transcutol® vendu par Gattefosse | 5,0 g |
| Triéthanolamine | 0,3 g |
| Conservateurs | 0,3 g |
| Propylène glycol | 3,0 g |
| NaOH | 0,007 g |
| Composé de l'exemple 4 | 0,1 g |
| Eau q.s.p. | 100 g |

| **Crème aux liposomes non ioniques** | |
|---|---|
| Carbopol 940® vendu par Goodrich | 0,2 g |
| Transcutol® vendu par Gattefosse | 3,0 g |
| Triéthanolamine | 0,2 g |
| Conservateurs | 0,3 g |
| Polyglycéryl 3 cétyl éther | 3,8 g |
| β sitostérol | 3,8 g |
| Dicétyl phosphate | 0,4 g |
| NaOH | 0,007 g |
| Composé de l'exemple 5 | 0,15 g |
| Huile de tournesol | 35,0 g |
| Parfum | 0,3 g |
| Eau q.s.p. | 100 g |

### EXEMPLE 8

Il a été réalisé un test comparatif des effets sur le lissage du cheveu d'une solution contenant le composé de l'exemple 4 à 1 % dans le tétrahydrofurane (THF), par rapport à un témoin constitué de THF, ou à une solution contenant, à la place du composé selon l'invention, soit l'isomère pur (forme D-ribo) correspondant au composé de l'exemple 4 (composé A), soit le composé de l'exemple 1 de la demande de brevet européen publiée sous le n°0 500 437 déposée par la Demanderesse (composé B).

Ce test détermine le coefficient de frottement des cheveux par mesure de la force à appliquer à une masse témoin pour la faire glisser à vitesse constante sur deux cheveux tendus parallèlement. La mesure est effectuée en faisant glisser la masse de la racine vers la pointe des cheveux (R->P) et inversement (P->R).
Les résultats sont rassemblés dans le tableau 1 suivant.

**Tableau 1**

| Composition | Traitement | Cheveux naturels | | Cheveux décolorés | |
|---|---|---|---|---|---|
| | | R->P | P->R | R->P | P->R |
| THF | non rincé | 0,186 ± 0,006 | 0,246 ± 0,004 | 0,181 ± 0,003 | 0,235 ± 0,007 |
| THF | rincé | 0,151 ± 0,006 | 0,220 ± 0,006 | 0,188 ± 0,006 | 0,256 ± 0,004 |
| composé B | non rincé | 0,130 ± 0,003 | 0,225 ± 0,005 | 0,136 ± 0,006 | 0,217 ± 0,008 |
| composé B | rincé | 0,106 ± 0,006 | 0,179 ± 0,005 | 0,119 ± 0,006 | 0,202 ± 0,006 |
| composé A | non rincé | 0,000 | 0,000 | 0,000 | 0,000 |
| composé A | rincé | 0,125 ± 0,004 | 0,214 ± 0,004 | 0,191 ± 0,004 | 0,264 ± 0,012 |
| exemple 4 | non rincé | 0,089 ± 0,003 | 0,143 ± 0,003 | 0,009 ± 0,006 | 0,147 ± 0,006 |
| exemple 4 | rincé | 0,095 ± 0,204 | 0,147 ± 0,004 | 0,085 ± 0,002 | 0,155 ± 0,004 |

L'application du composé selon l'invention (exemple 4) permet une nette diminution du coefficient de frottement, démontrant ainsi une amélioration du lissage ou du démêlage des cheveux.
Le composé A, qui correspond à l'isomère pur, se dépose sur le cheveu (pas de possibilité de lissage) et rend ainsi impossible la mesure selon ce test lors d'un traitement non rincé, ce résultat montre donc un des inconvénients des produits sous forme d'un seul isomère.

### EXEMPLE 9

### Mesure de la perte insensible en eau (PIE)

Cette mesure s'effectue à l'aide d'un évaporimètre (Servomed) qui détermine quantitativement l'évaporation d'eau, c'est-à-dire un transport d'eau par diffusion, à partir d'un échantillon de stratum corneum préalablement délipidé obturant une capsule cylindrique contenant de l'eau, le tout étant placé dans une chambre à température et humidité relatives contrôlées.
Des capteurs permettent de mesurer la pression partielle de vapeur d'eau en deux points situés à des distances différentes de l'échantillon.
On détermine ainsi le gradient de pression partielle de vapeur d'eau entre les deux points, et donc le taux d'évaporation conformément à la loi de Fick.
Il a été réalisé un test comparatif des effets sur la P.l.E. d'une solution contenant le composé de l'exemple 2 ou 4 dans le tétrahydrofuranne (THF), par rapport à une solution contenant, à la place du composé selon l'invention, soit l'isomère pur (forme D-ribo) correspondant au composé de l'exemple 4 (composé A), soit le composé de l'exemple 1 de la demande de brevet européen publiée sous le n°0 500 437 déposée par la Demanderesse (composé B).
Les résultats sont rassemblés dans le tableau 2 suivant.

**Tableau 2**

| Composé | Composition (concentration) | P.I.E. 20 H (%) |
|---|---|---|
| Composé B | 1,5% dans THF | -8 ± 1 |
| Exemple 4 | 1,5% dans THF | -15 ± 2 |
| Composé A | 1% dans THF | 0 ± 2 |
| Exemple 4 | 1% dans THF | -9 ± 2 |
| Composé B | 2% dans THF | -17 ± 3 |
| Exemple 2 | 2% dans THF | -31 ± 2 |

On constate donc que l'application des composés selon l'invention permet de réduire de manière significative l'évaporation de l'eau contenue dans le stratum corneum, démontrant ainsi des propriétés améliorées pour les composés selon l'invention de barrière de perméabilité à l'eau de stratum corneum

## Revendications

1. Composés répondant à la formule : 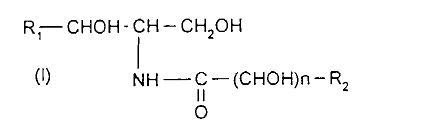 dans laquelle :
* R₁ désigne un radical alkyle hydroxylé, saturé ou insaturé, en C₁₀ à C₂₅ ;
* n est égal à 0 ou 1; ;
* R₂ désigne un radical alkyle linéaire ou ramifié, saturé ou insaturé, en C₁ à C₃₁, lorsque n égal 1; R₂ désigne un radical alkyle linéaire ou ramifié, saturé ou insaturé, en C₂ à C₃₁, lorsque n égal 0 ;
les composés étant sous forme de mélanges de steréoisomères au moins sur la partie amino-alcool de ladite formule (I).

2. Composés selon la revendication 1, caractérisés en ce que, lorsque R₁ désigne un radical alkyle saturé et hydroxylé, le radical hydroxyle se trouve en position α par rapport au carbone du groupement -CHOH-.

3. Composés selon la revendication 1, caractérisés en ce que, lorsque R₁ désigne un radical alkyle insaturé et hydroxylé, R₁ présente un radical éthylénique en position α par rapport au carbone du groupement -CHOH-.

4. Composés selon la revendication précédente, caractérisé en ce qu'au moins un, et de préférence le, radical hydroxyle est en position α du radical éthylénique.

5. Composés selon l'une quelconque des revendications précédentes, caractérisés en ce que R₁ désigne un radical alkyle en C₁ à C₂₃.

6. Composés selon l'une des revendications précédentes, caractérisés en ce que R₂ désigne un radical alkyle linéaire, plus particulièrement en C₂ à C₂₅.

7. Composés selon l'une quelconque des revendications précédentes, caractérisés en ce qu'ils sont sous forme d'au moins 4 isomères.

8. Composés selon l'une quelconque des revendications précédentes, caractérisés en ce qu'ils sont choisis parmi le 2-N-docosanoylamino-octadécane-1,3,4-triol, le 2-N-(2-hydroxy-hexadecanoyl)-amino-octadécane-1,3,4-triol et le 2-N-hexadecanoylaminooctadécane-1 ,3,4-triol.

9. Procédé de préparation de composés de formule (I) selon la revendication 1, caractérisé en ce qu'il comprend :
(a) l'acylation d'au moins un composé de formule (II) suivante : 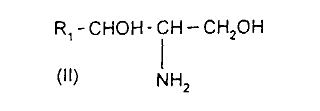 sous forme de mélange d'au moins 2 isomères,
formule (II) dans laquelle R₁ a la signification donnée dans les revendications précédentes, ou d'au moins un dérivé réactif de ces composés de formule (II), à l'aide d'au moins un agent acylant de formule (III) suivante :
R₂-(CHX)ₙ-CO-A (III)
formule (III) dans laquelle R₂ et n ont les significations données dans les revendications précédentes et X représente un groupe choisi parmi le radical hydroxyle, un atome d'halogène, de préférence Br, Cl ou I, et un radical de formule OB susceptible de former un groupe OH ; A est un groupe choisi parmi un atome d'halogène, un radical de formule -O-COO-R₅, un radical de formule OR₆, un groupement 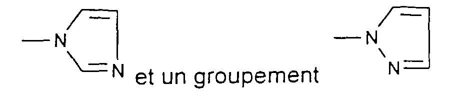 où R₅ est un radical alkyle inférieur en C₂ à C₈ et R₆ est un groupe choisi parmi un radical alkyle inférieur en C₁ à C₈, 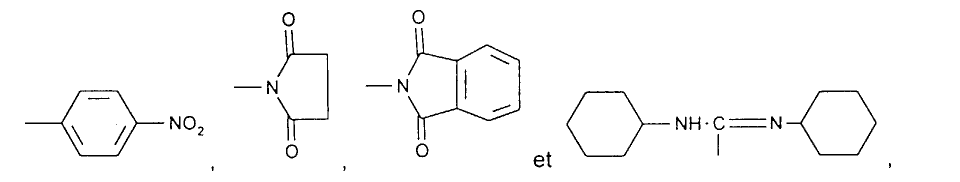
(b) l'isolation des composés obtenus, et
(c) éventuellement, lorsque X est différent du radical hydroxyle, l'hydrolyse ou l'hydrogénolyse des composés obtenus pour transformer le groupe OB en radical hydroxyle, précédé éventuellement, lorsque X est un atome d'halogène, d'une étape de substitution de X par un groupe OB.

10. Procédé selon la revendication précédente, caractérisé en ce qu'il comprend lorsque X est le radical hydroxyle, après l'étape (a) d'acylation et avant l'étape (b) d'isolation du composé de fomule (I) obtenu, une étape de protection des groupements hydroxyle, par réaction avec un agent choisi parmi les anhydrides d'acide, les halogénures d'acide et les chlorosilanes, la réaction étant suivie, après isolement des composés selon l'étape (b), par une hydrolyse ou une hydrogénolyse.

11. Procédé selon la revendication précédente, caractérisé en ce que les agents sont choisis parmi l'anhydride acétique, le chlorure de benzoyle, le chlorure de benzyle, le bromure de benzyle, les chlorosilanes de formule CISi(CH₃)₃, CISi(CH₃)₂(tBu), CISi(tBu)(-C₆H₅)₂.

12. Procédé selon l'une des revendications 9 à 11, caractérisé en ce que le radical OB de l'agent acylant est choisi parmi les radicaux suivants : acétate, benzoate, benzyloxy, -OSi(CH₃)₃, -OSi(CH₃)₂(t-butyl), et -OSi(t-butyl)(-C₆H₅)₂.

13. Procédé selon l'une des revendications 9 à 12, caractérisé en ce que le procédé est mis en oeuvre dans un solvant choisi parmi le tétrahydrofurane, la pyridine, le 1,2-diméthoxy-éthane, le diméthylformamide, le dichlorométhane et le tertiobutylméthyléther.

14. Procédé selon l'une des revendications 9 à 13, caractérisé en ce que le groupe A de l'agent acylant de formule (III) est choisi parmi les goupes suivants :
-Cl, un radical de formule -O-COO-C₂H₅, un radical de formule -OCH₃ ou -OC₂H₅,

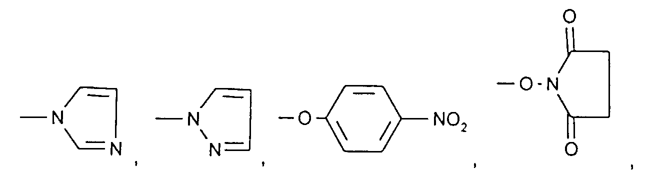

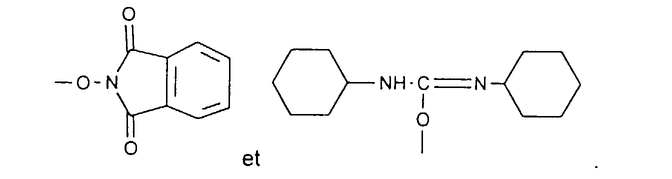

15. Composition à usage cosmétique ou dermatologique, caractérisée par le fait qu'elle contient de 0,005 à 20 % en poids, de préférence de 0,01 à 10 % en poids, de composé de formule (I) selon l'une des revendications 1 à 8 en présence d'un adjuvant choisi parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

16. Composition selon la revendication précédente, caractérisée par le fait qu'elle se présente sous forme d'émulsion dont la phase grasse, représentant 5 à 60 % du poids total de l'émulsion, est essentiellement constituée d'un mélange de composé de formule (I) avec au moins une huile, la phase aqueuse constituant 30 à 85 % du poids total de l'émulsion, l'agent émulsionnant étant présent à raison de 1 à 20 % en poids, et de préférence 2 à 12 % en poids par rapport au poids total de l'émulsion.

17. Composition selon la revendication 15, caractérisée par le fait qu'elle se présente sous forme de lotion huileuse, oléoalcoolique ou hydroalcoolique, sous forme de gel, de dispersion ou de bâtonnets solides, de spray ou de mousse aérosol.

18. Composition selon la revendication 17, caractérisée par le fait qu'elle se présente sous forme d'une dispersion aqueuse de sphérules lipidiques, constituées de couches moléculaires organisées renfermant une phase aqueuse encapsulée, ces couches étant constituées d'au moins un composé de formule (I) associé à au moins un autre composé lipidique.

19. Composition sous forme de dispersion aqueuse de sphérules lipidiques selon la revendication 18, caractérisée par le fait que l'autre composé lipidique est choisi parmi les alcools et diols à longue chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras, les alkylsulfates et les acides gras sous forme de sels.

20. Composition sous forme de dispersion aqueuse de sphérules lipidiques selon la revendication 18 ou 19, caractérisée par le fait que les sphérules ont un diamètre compris entre 0,05 µm et 5 µm.

21. Utilisation du composé de formule (I) selon l'une quelconque des revendications 1 à 8, dans des émulsions, des dispersions, des gels, des bâtonnets solides, des vernis ou dans des lotions à usage cosmétique ou dermatologique.

22. Utilisation du composé de formule (I) selon l'une quelconque des revendications 1 à 8 en association avec au moins un autre composé lipidique, pour la formation de dispersions de sphérules lipidiques à usage cosmétique ou dermatologique.

23. Procédé de traitement cosmétique hydratant de la peau, des cheveux, des ongles ou des cils, caractérisé par le fait qu'il consiste à appliquer sur la peau, les cheveux, les ongles ou les cils, une quantité suffisante d'au moins un composé selon l'une quelconque des revendications 1 à 8.

## Patentansprüche

1. Verbindungen der folgenden Formel: 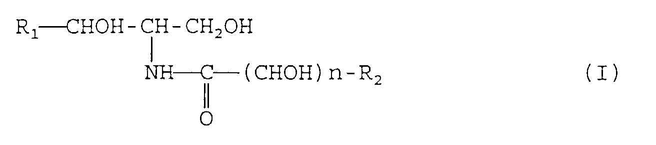 worin bedeuten:
. R₁ eine gesättigte oder ungesättigte hydroxyhaltige Alkylgruppe mit 10 bis 25 Kohlenstoffatomen;
. n Null oder 1;
. R₂
eine geradkettige oder verzweigte, gesättigte oder ungesättigte Alkylgruppe mit 1 bis 31 Kohlenstoffatomen, wenn n 1 ist;
eine geradkettige oder verzweigte, gesättigte oder ungesättigte Alkylgruppe mit 2 bis 31 Kohlenstoffatomen, wenn n Null ist;
wobei die Verbindungen in Form von Gemischen von Stereoisomeren zumindest bezüglich des Aminoalkoholteils der Formel (1) vorliegen.

2. Verbindungen nach Anspruch 1, dadurch gekennzeichnet, daß sich die Hydroxygruppe bezogen auf den Kohlenstoff der Gruppe -CHOH- in α-Stellung befindet, wenn R₁ eine gesättigte hydroxyhaltige Alkylgruppe bedeutet.

3. Verbindungen nach Anspruch 1, dadurch gekennzeichnet, daß R₁ eine ethylenisch ungesättigte Gruppe in α-Stellung zum Kohlenstoffatom der Gruppe -CHOH- aufweist, wenn R₁ eine ungesättigte hydroxyhaltige Alkylgruppe bedeutet.

4. Verbindungen nach dem vorhergehenden Anspruch, dadurch gekennzeichnet, daß sich mindestens eine und vorzugsweise die Hydroxygruppe in α-Stellung zur ethylenisch ungesättigten Gruppe befindet.

5. Verbindungen nach einem der vorhergehenden Ansprüche, dadurch gekennzeichnet, daß R₁ eine C₁₂₋₂₃-Alkylgruppe ist.

6. Verbindung nach einem der vorhergehenden Ansprüche, dadurch gekennzeichnet, daß R₂ eine geradkettige Alkylgruppe mit insbesondere 2 bis 25 Kohlenstoffatomen bedeutet.

7. Verbindungen nach einem der vorhergehenden Ansprüche, dadurch gekennzeichnet, daß sie in Form von mindestens 4 Isomeren vorliegen.

8. Verbindungen nach einem der vorhergehenden Ansprüche, dadurch gekennzeichnet, daß sie unter 2-N-Docosanoylamino-octadecan-1,3,4-triol, 2-N-(2-Hydroxy-hexadecanoyl)-amino-octadecan-1,3,4-triol und 2-N-Hexadecanoylamino-octadecan-1,3,4-triol ausgewählt sind.

9. Verfahren zur Herstellung von Verbindungen der Formel (I) nach Anspruch 1, dadurch gekennzeichnet, daß es die folgenden Schritte umfaßt:
(a) Acylierung mindestens einer Verbindung der folgenden Formel II: 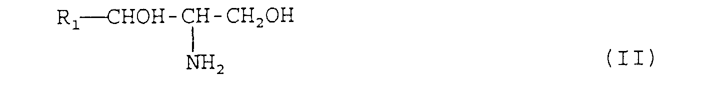 in Form des Gemisches von mindestens 2 Isomeren, wobei in Formel (II) R₁ die oben in den vorhergehenden Ansprüchen angegebene Bedeutung aufweist,
oder Acylierung mindestens eines reaktiven Derivats der Verbindungen der Formel (II),
mit mindestens einem Acylierungsmittel der folgenden Formel (III):
R₂-(CHX)ₙ-CO-A (III)
wobei in Formel (III) R₂ und n die oben in den vorhergehenden Ansprüchen angegebenen Bedeutungen aufweisen, X unter Hydroxy, Halogen, vorzugsweise Br, Cl oder I, und einer Gruppe der Formel OB, welche befähigt ist, eine OH-Gruppe zu bilden, ausgewählt ist, und A eine Gruppe bedeutet, die unter Halogen, einer Gruppe der Formel -O-COO-R₅, einer Gruppe der Formel OR₆, der Gruppe 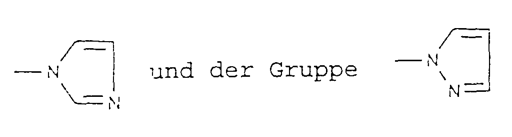 ausgewählt ist, wobei R₅ eine niedere C₂₋₈-Alkylgruppe ist und R₆ eine Gruppe bedeutet, die unter einer niederen C₁₋₈-Alkylgruppe, 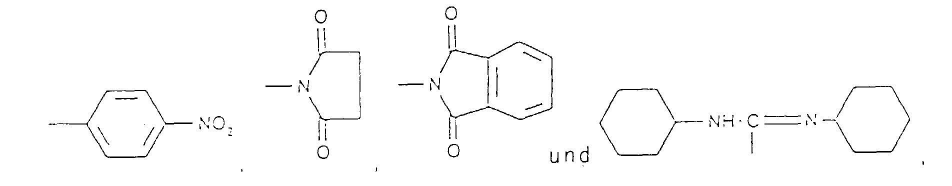 ausgewählt ist;
(b) Abtrennung der so hergestellten Verbindungen, und
(c) gegebenenfalls Hydrolyse oder Hydrogenolyse der erhaltenen Verbindungen, wenn X von Hydroxy verschieden ist, um die Gruppe OB in eine Hydroxygruppe umzuwandeln, wobei zuvor gegebenenfalls die Gruppe X durch eine Gruppe OB substituiert wird, wenn X ein Halogenatom ist.

10. Verfahren nach dem vorhergehenden Anspruch, dadurch gekennzeichnet, daß es, wenn X Hydroxy bedeutet, nach Schritt (a) der Acylierung und vor Schritt (b) der Abtrennung der hergestellten Verbindung der Formel (I) einen Schritt umfaßt, in dem die Hydroxygruppen durch Umsetzung mit einem Mittel geschützt wird, das unter den Säureanhydriden, Säurehalogeniden und Chlorsilanen ausgewählt ist, wobei nach der Abtrennung der Verbindungen gemäß Schritt (b) eine Hydrolyse oder Hydrogenolyse durchgeführt wird.

11. Verfahren nach dem vorhergehenden Anspruch, dadurch gekennzeichnet, daß die Mittel unter Essigsäureanhydrid, Benzoylchlorid, Benzylchlorid, Benzylbromid und den Chlorsilanen der Formel ClSi(CH₃)₃, ClSi(CH₃)₂(tBu) und ClSi(tBu)(-C₆H₅)₂ ausgewählt sind.

12. Verfahren nach einem der Ansprüche 9 bis 11, dadurch gekennzeichnet, daß die Gruppe OB des Acylierungsmittels unter den folgenden Gruppen ausgewählt ist: Acetat, Benzoat, Benzyloxy, OSi(CH₃)₃, -OSi(CH₃)₂(t-butyl) und -OSi(t-butyl) (-C₆H₅)₂.

13. Verfahren nach einem der Ansprüche 9 bis 12, dadurch gekennzeichnet, daß das Verfahren in einem Lösungsmittel durchgeführt wird, das unter Tetrahydrofuran, Pyridin, 1,2-Dimethoxyethan, Dimethylformamid, Dichlormethan und tert.-Butylmethylether ausgewählt ist.

14. Verfahren nach einem der Ansprüche 9 bis 13, dadurch gekennzeichnet, daß die Gruppe A des Acylierungsmittels der Formel (III) unter den folgenden Gruppen ausgewählt ist:
-Cl, -O-COO-C₂H₅, -OCH₃, -OC₂H₅,

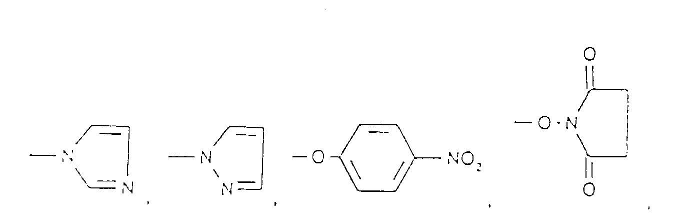

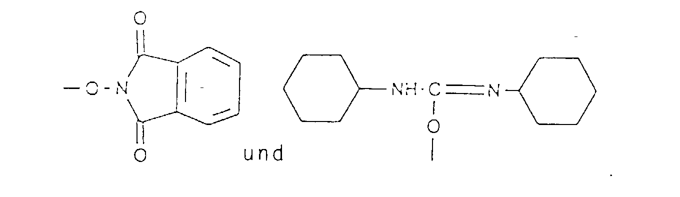

15. Zusammensetzung zur kosmetischen oder dermatologischen Verwendung, dadurch gekennzeichnet, daß sie 0,005 bis 20 Gew.-% und vorzugsweise 0,01 bis 10 Gew.-% einer Verbindung der Formel (I) nach einem der Ansprüche 1 bis 8 in Gegenwart eines Hilfsstoffes enthält, der unter den Fettsubstanzen, Lösungsmitteln, Wasser, Verdikkungsmitteln, Emulgatoren, hydratisierenden Produkten, reizlindernden Mitteln, Sonnenschutzfiltern, keimtötenden Mitteln, Färbemitteln, Konservierungsmitteln, Parfums, Treibmitteln und/oder grenzflächenaktiven Stoffen ausgewählt ist.

16. Zusammensetzung nach dem vorhergehenden Anspruch, dadurch gekennzeichnet, daß sie in Form einer Emulsion vorliegt, deren Fettphase 5 bis 60 % des Gesamtgewichts der Emulsion ausmacht und im wesentlichen aus einem Gemisch der Verbindung der Formel (I) mit mindestens einem Öl besteht, wobei die wäßrige Phase 30 bis 85 % des Gesamtgewichts der Emulsion ausmacht und der Emulgator in einem Mengenanteil von 1 bis 20 Gew.-% und vorzugsweise 2 bis 12 Gew.-%, bezogen auf das Gesamtgewicht der Emulsion, vorliegt.

17. Zusammensetzung nach Anspruch 15, dadurch gekennzeichnet, daß sie in Form einer öligen, ölig-alkoholischen oder wäßrig-alkoholischen Lotion, in Form eines Gels, einer Dispersion oder in Form von festen Stiften, als Spray oder Aerosolschaum vorliegt.

18. Zusammensetzung nach Anspruch 17, dadurch gekennzeichnet, daß sie in Form einer wäßrigen Dispersion von Lipidkügelchen vorliegt, die aus organisierten molekularen Schichten bestehen, welche eine wäßrige Phase einkapseln, wobei die Schichten aus mindestens einer Verbindung der Formel (I) in Kombination mit mindestens einer weiteren Lipidverbindung bestehen.

19. Zusammensetzung in Form einer wäßrigen Dispersion von Lipidkügelchen nach Anspruch 18, dadurch gekennzeichnet, daß die weitere Lipidverbindung unter den langkettigen Alkoholen und Diolen, Sterinen, Phospholipiden, Glykolipiden, Cholesterylsulfat, Cholesterylphosphat, langkettigen Aminen und deren quaternären Ammoniumderivaten, Dihydroxyalkylaminen, polyethoxylierten Fettaminen, Estern von langkettigen Aminoalkoholen, deren Salzen und quaternären Ammoniumderivaten, Phosphorsäureestern mit Fettalkoholen, Alkylsulfaten und Fettsäure in Form von Salzen ausgewählt ist.

20. Zusammensetzung in Form einer wäßrigen Dispersion von Lipidkügelchen nach Anspruch 18 oder 19, dadurch gekennzeichnet, daß die Kügelchen einen Durchmesser im Bereich von 0,05 bis 5 µm aufweisen.

21. Verwendung der Verbindung der Formel (I) nach einem der Ansprüche 1 bis 8 in Emulsionen, Dispersionen, Gelen, festen Stiften, Lacken oder Lotionen zur kosmetischen oder dermatologischen Anwendung.

22. Verwendung der Verbindung der Formel (I) nach einem der Ansprüche 1 bis 8 in Kombination mit mindestens einer weiteren Lipidverbindung zur Bildung von Dispersionen von Lipidkügelchen zur kosmetischen oder dermatologischen Anwendung.

23. Verfahren zur kosmetischen hydratisierenden Behandlung der Haut, der Haare, der Nägel oder der Wimpern, dadurch gekennzeichnet, daß es darin besteht, auf die Haut, die Haare, die Nägel oder die Wimpern eine wirksame Menge mindestens einer Verbindung nach einem der Ansprüche 1 bis 8 aufzutragen.

## Claims

1. Compounds corresponding to the formula: 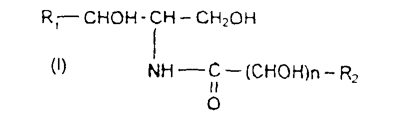 in which:
* R₁ denotes a saturated or unsaturated C₁₀ to C₂₅ hydroxylated alkyl radical;
* n is equal to 0 or 1;
* R₂ denotes a linear or branched, saturated or unsaturated C₁ to C₃₁ alkyl radical, when n is equal to 1; R₂ denotes a linear or branched, saturated or unsaturated C₂ to C₃₁ alkyl radical, when n is equal to 0;
the compounds being in the form of mixtures of stereoisomers at least on the amino alcohol part of the said formula (I).

2. Compounds according to Claim 1, characterized in that, when R₁ denotes a saturated and hydroxylated alkyl radical, the hydroxyl radical is in an α position relative to the carbon of the -CHOH- group.

3. Compounds according to Claim 1, characterized in that, when R₁ denotes an unsaturated and hydroxylated alkyl radical, R₁ has an ethylenic radical in an α position relative to the carbon of the -CHOH-group.

4. Compounds according to the preceding claim, characterized in that at least one, and preferably the, hydroxyl radical is in a position α to the ethylenic radical.

5. Compounds according to any one of the preceding claims, characterized in that R₁ denotes a C₁₂ to C₂₃ alkyl radical.

6. Compounds according to one of the preceding claims, characterized in that R₂ denotes a linear alkyl, more particularly C₂ to C₂₅ alkyl, radical.

7. Compounds according to any one of the preceding claims, characterized in that they are in the form of at least 4 isomers.

8. Compounds according to any one of the preceding claims, characterized in that they are chosen from 2-N-docosanoylaminooctadecane-1,3,4-triol, 2-N-(2-hydroxyhexadecanoyl)aminooctadecane-1,3,4-triol and 2-N-hexadecanoylaminooctadecane-1,3,4-triol.

9. Process for the preparation of compounds of formula (I) according to Claim 1, characterized in that it comprises:
(a) the acylation of at least one compound of following formula (II): 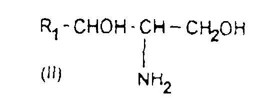 in the form of a mixture of at least 2 isomers,
in which formula (II) R₁ has the meaning given in the preceding claims, or of at least one reactive derivative of these compounds of formula (II), using at least one acylating agent of following formula (III):
R₂- (CHX)ₙ-CO-A (III)
in which formula (III) R₂ and n have the meanings given in the preceding claims and X represents a group chosen from the hydroxyl radical, a halogen atom, preferably Br, Cl or I, and a radical of formula OB capable of forming an OH group; A is a group chosen from a halogen atom, a radical of formula -O-COO-R₅, a radical of formula OR₆, a group 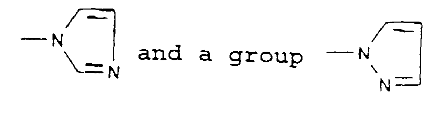 where R₅ is a C₂ to C₈ lower alkyl radical and R₆ is a group chosen from a C₁ to C₈ lower alkyl radical, 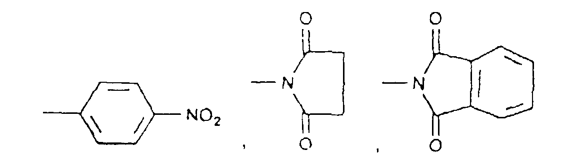 and 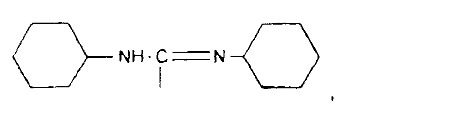
(b) the isolation of the compounds obtained, and
(c) optionally, when X is other than a hydroxyl radical, the hydrolysis or hydrogenolysis of the compounds obtained in order to convert the group OB into a hydroxyl radical, optionally preceded, when X is a halogen atom, by a step of substitution of X with a group OB.

10. Process according to the preceding claim, characterized in that it comprises when X is the hydroxyl radical, after the step (a) of acylation and before the step (b) of isolation of the compound (I) obtained, a step for protection of the hydroxyl groups, by reaction with an agent chosen from acid anhydrides, acid halides and chlorosilanes, the reaction being followed, after isolation of the compounds according to the step (b), by a hydrolysis or a hydrogenolysis.

11. Process according to the preceding claim, characterized in that the agents are chosen from acetic anhydride, benzoyl chloride, benzyl chloride, benzyl bromide and the chlorosilanes of formulae ClSi(CH₃)₃, ClSi(CH₃)₂(tBu) and ClSi(tBu)(-C₆H₅)₂.

12. Process according to one of Claims 9 to 11, characterized in that the radical OB of the acylating agent is chosen from the following radicals: acetate, benzoate, benzyloxy, -OSi(CH₃)₃, -OSI(CH₃)₂(t-butyl), and -OSi(t-butyl) (-C₆H₅)₂.

13. Process according to one of Claims 9 to 12, characterized in that the process is carried out in a solvent chosen from tetrahydrofuran, pyridine, 1,2-dimethoxyethane, dimethylformamide, dichloromethane and tert-butyl methyl ether.

14. Process according to one of Claims 9 to 13, characterized in that the group A of the acylating agent of formula (III) is chosen from the following groups:
-Cl, a radical of formula -O-COO-C₂H₅, a radical of formula -OCH₃ or -OC₂H₅,

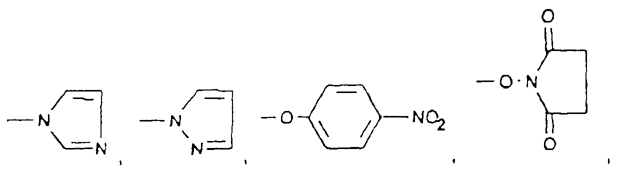

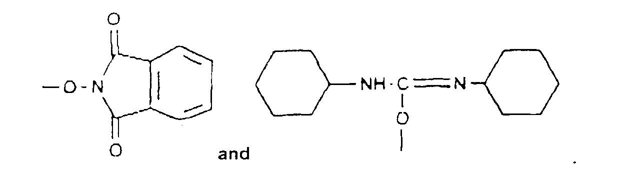

15. Composition for cosmetic or dermatological use, characterized in that it contains from 0.005 to 20% by weight, preferably from 0.01 to 10% by weight, of compound of formula (I) according to one of Claims 1 to 8 in the presence of an adjuvant chosen from fatty substances, solvents, water, thickening agents, emulsifying agents, moisturizing products, softeners, sunscreens, germicides, dyes, preserving agents, fragrances, propellants and surfactants.

16. Composition according to the preceding claim, characterized in that it is in the form of an emulsion whose fatty phase, representing 5 to 60% of the total weight of the emulsion, consists essentially of a mixture of a compound of formula (I) with at least one oil, the aqueous phase constituting 30 to 85% of the total weight of the emulsion, the emulsifying agent being present in a proportion of 1 to 20% by weight, and preferably 2 to 12% by weight, relative to the total weight of the emulsion.

17. Composition according to Claim 15, characterized in that it is in the form of an oily, oleo-alcoholic or aqueous-alcoholic lotion or in the form of a gel, a dispersion or solid sticks, a spray or an aerosol foam.

18. Composition according to Claim 17, characterized in that it is in the form of an aqueous dispersion of lipid spherules, consisting of organized molecular layers containing an encapsulated aqueous phase, these layers consisting of at least one compound of formula (I) associated with at least one other lipid compound.

19. Composition in the form of an aqueous dispersion of lipid spherules according to Claim 18, characterized in that the other lipid compound is chosen from long-chain alcohols and diols, sterols, phospholipids, glycolipids, cholesteryl sulphate and phosphate, long-chain amines and their quaternary ammonium derivatives, dihydroxyalkylamines, polyoxyethylenated fatty amines, long-chain amino alcohol esters, their salts and quaternary ammonium derivatives, phosphoric esters of fatty alcohols and alkyl sulphates, fatty acids in the form of salts.

20. Composition in the form of an aqueous dispersion of lipid spherules according to Claim 18 or 19, characterized in that the spherules have a diameter of between 0.05 µm and 5 µm.

21. Use of the compound of formula (I) according to any one of Claims 1 to 8, in emulsions, dispersions, gels, solid sticks, varnishes or in lotions for cosmetic or dermatological use.

22. Use of the compound of formula (I) according to any one of Claims 1 to 8, in combination with at least one other lipid compound, for the formation of dispersions of lipid spherules for cosmetic or dermatological use.

23. Process for the moisturizing cosmetic treatment of the skin, the hair, the nails or the eyelashes, characterized in that it consists in applying a sufficient amount of at least one compound according to any one of Claims 1 to 8 to the skin, the hair, the nails or the eyelashes.
